(19) Europäisches Patentamt
European Patent Office
Office européen des brevets

(11) Veröffentlichungsnummer: 0 290 902
A2

(12) # EUROPÄISCHE PATENTANMELDUNG

(21) Anmeldenummer: 88107009.8

(22) Anmeldetag: 02.05.88

(51) Int. Cl.4: C07D 513/04 , A01N 43/90 , C07C 119/048 , C07C 161/04 , //(C07D513/04,277:00,235:00)

(30) Priorität: 14.05.87 DE 3716108
27.11.87 DE 3740256

(43) Veröffentlichungstag der Anmeldung:
17.11.88 Patentblatt 88/46

(84) Benannte Vertragsstaaten:
BE CH DE FR GB IT LI NL

(71) Anmelder: BAYER AG
Konzernverwaltung RP Patentabteilung
D-5090 Leverkusen 1 Bayerwerk(DE)

(72) Erfinder: Lindel, Hans, Dr.
Carl-Duisberg-Strasse 321
D-5090 Leverkusen 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Unterdorfstrasse 6A
D-4000 Düsseldorf 31(DE)

(54) **Anellierte (Thio)Hydantoine.**

(57) Die Erfindung betrifft neue anellierte (Thio)Hydantoine der Formel

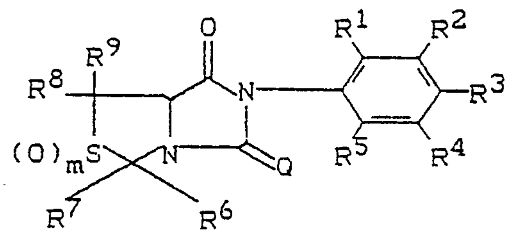

(I)

in welcher $R^1$ bis $R^9$, Q und m die in der Beschreibung angegebene Bedeutung haben, ein Verfahren zu ihrer Herstellung und ihre Verwendung als Herbizide.

EP 0 290 902 A2

## Anellierte (Thio)Hydantoine

Die vorliegende Erfindung betrifft neue anellierte (Thio)Hydantoine, ein Verfahren zu ihrer Herstellung und ihre Verwendung als Herbizide.

Es ist bereits bekannt geworden, daß bestimmte heterocyclische Imide, wie z. B. 2-(4-Chlor-2-fluor-5-methoxycarbonylmethoxy-phenyl)-4,5,6,7-tetrahydro-2H-isoindol-1,3-dion, als Herbizide verwendet werden können (vgl. EP-A 83 055). Die Wirkung der bekannten Verbindungen ist jedoch nicht immer zufriedenstellend.

Weiter ist bekannt geworden, daß andere heterocyclische Imide fungizid wirksam sind (vgl. JP-A 79-157590, zit. in Chem. Abstracts 93 (1980), 46682x). In der Literatur sind weitere heterocyclische Imide beschrieben (vgl. C.A. 99: 70655k (1983), C.A. 105: 115004h und 202732n (1986)), welche bei der Beschreibung der neuen anellierten Thio(Hydantoine) durch einen Disclaimer ausgeschlossen sind. Eine herbizide Wirksamkeit dieser Verbindungen ist jedoch nicht bekannt.

Es wurden nun neue anellierte (Thio)Hydantoine der allgemeinen Formel (I)

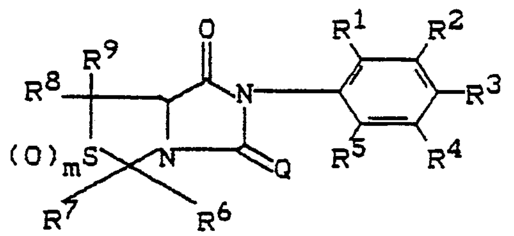

(I)

in welcher

m für die Zahlen 0, 1 oder 2 steht,

$R^1$ für Wasserstoff oder Halogen steht,

$R^2$ für Wasserstoff, Halogen, Nitro, Cyan oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht,

$R^3$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht oder die beiden Reste

$R^2$ und $R^3$ zusammen für die Gruppierung $-X-A-(CO)_n-Y-$stehen, worin

n für die Zahlen 0 oder 1 steht,

A für eine direkte Bindung oder für geradkettiges oder verzweigtes und gegebenenfalls durch Halogen substituiertes Alkandiyl steht,

X für Sauerstoff oder Schwefel steht und

Y für Sauerstoff, Schwefel oder die Gruppierung $N-R^{10}$ steht, worin

$R^{10}$ für Wasserstoff oder gegebenenfalls substituiertes Alkyl, Alkenyl oder Alkinyl steht,

in welcher weiter

$R^4$ für Wasserstoff oder Halogen steht,

$R^5$ für Wasserstoff oder Halogen steht,

$R^6$ für Wasserstoff, gegebenenfalls substituiertes Alkyl oder gegebenenfalls substituiertes Phenyl oder Naphthyl steht,

$R^7$ für Wasserstoff oder Alkyl steht,

$R^8$ für Wasserstoff oder Alkyl steht,

$R^9$ für Wasserstoff oder Alkyl steht und

Q für Sauerstoff oder Schwefel steht

-mit der Maßgabe, daß

1. wenigstens einer der Reste $R^1$ bis $R^9$ von Wasserstoff verschieden ist,
2. $R^3$ nur dann für Methyl, Methoxy oder Chlor steht, wenn wenigstens einer der Reste $R^1$, $R^2$, $R^4$ und $R^5$ von Wasserstoff verschieden ist und

3. die Reste $R^2$ und $R^4$ nur dann gleichzeitig für Chlor stehen, wenn wenigstens einer der Reste $R^1$, $R^3$ und $R^5$ von Wasserstoff verschieden ist -
gefunden.

Die Erfindung betrifft sowohl die einzelnen möglichen Stereoisomeren der Formel (I) als auch die Gemische dieser Isomeren.

Weiter wurde gefunden, daß man die neuen anellierten (Thio)Hydantoine der allgemeinen Formel (I) erhält, wenn man Aryliso(thio)cyanate der allgemeinen Formel (II)

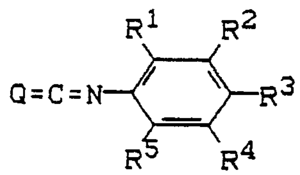

(II)

in welcher
$R^1$, $R^2$, $R^3$, $R^4$, $R^5$ und Q die oben angegbenen Bedeutungen haben,
mit Thiazolidincarbonsäure(ester)n der allgemeinen Formel (III)

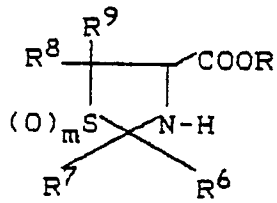

(III)

in welcher
m, $R^6$, $R^7$, $R^8$ und $R^9$ die oben angegebenen Bedeutungen haben und
R für Wasserstoff oder Alkyl steht,
gegebenenfalls in Gegenwart eines Säureakzeptors und gegebenenfalls in Gegenwart eines Verdünnungsmittels umsetzt.

Ein weiterer Syntheseweg zu den anellierten (thio)Hydantionen der allgemeinen Formel (I) ist nachstehend skizziert, wobei $R^1$ bis $R^9$ und Q die oben angegebenen Bedeutungen haben:

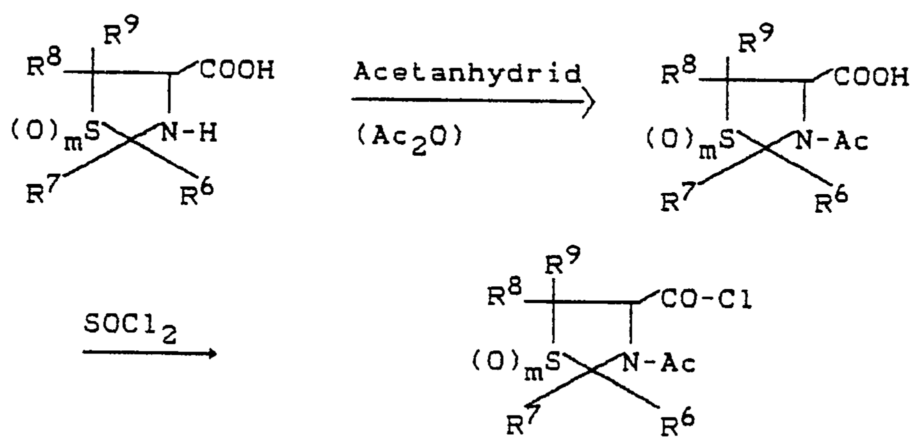

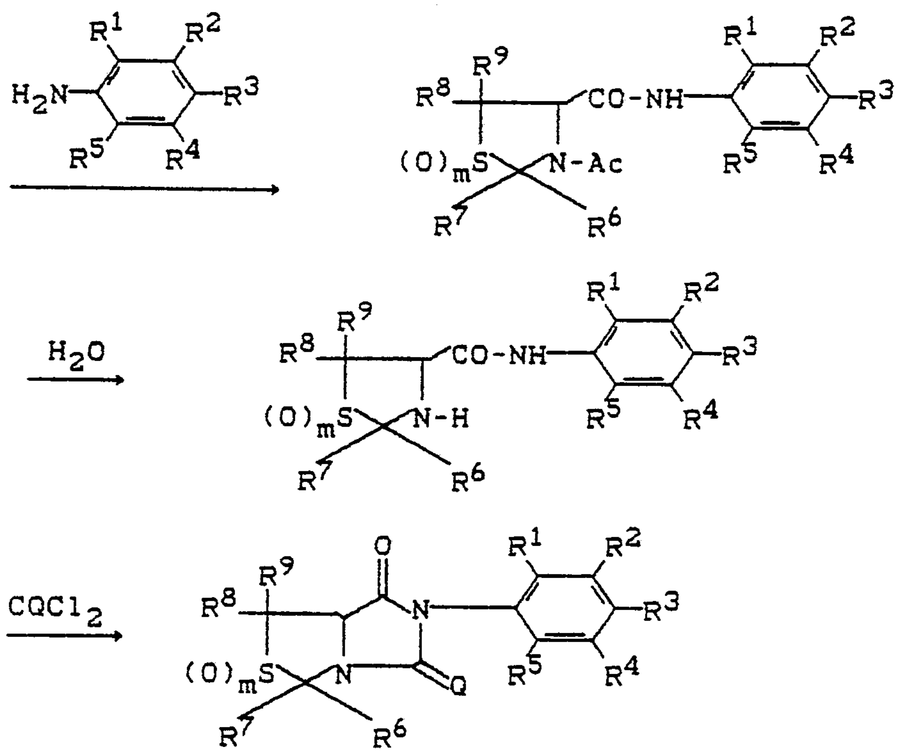


Schließlich wurde gefunden, daß die neuen anellierten (Thio)Hydantoine der allgemeinen Formel (I) hervorragende herbizide Eigenschaften aufweisen. Ebenso zeigen die bekannten anellierten (Thio)-Hydantoine (vgl. Disclaima) eine sehr gute herbizide Wirksamkeit.

Die Erfindung betrift also die Verwendung der erfindungsgemäßen Verbindungen der Formel (I) und der bekannten anellierten (Thio)Hydantoine, welche in der folgenden Formel (Ia) zusammengefaßt sind:

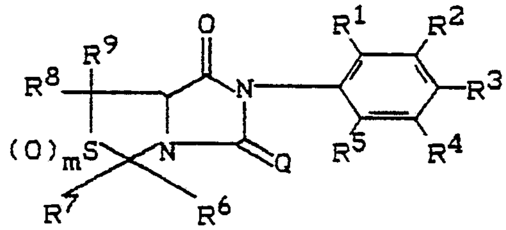

(Ia)

in welcher

m für die Zahlen 0, 1 oder 2 steht,

$R^1$ für Wasserstoff oder Halogen steht,

$R^2$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht,

$R^3$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht oder die beiden Reste

$R^2$ und $R^3$ zusammen für die Gruppierung $-X-A-(CO)_n-Y-$stehen, worin

n für die Zahlen 0 oder 1 steht,

A für eine direkte Bindung oder für geradkettiges oder verzweigtes und gegebenenfalls durch Halogen substituiertes Alkandiyl steht,

X für Sauerstoff oder Schwefel steht und

Y für Sauerstoff, Schwefel oder die Gruppierung $N-R^{10}$ steht, worin

$R^{10}$ für Wasserstoff oder gegebenenfalls substituiertes Alkyl, Alkenyl oder Alkinyl steht,

in welcher weiter

$R^4$ für Wasserstoff oder Halogen steht,

$R^5$ für Wasserstoff oder Halogen steht,

$R^6$ für Wasserstoff, gegebenenfalls substituiertes Alkyl oder gegebenenfalls substituiertes Phenyl oder Naphthyl steht,

$R^7$ für Wasserstoff oder Alkyl steht,

$R^8$ für Wasserstoff oder Alkyl steht,

$R^9$ für Wasserstoff oder Alkyl steht und

Q für Sauerstoff oder Schwefel steht.

Überraschenderweise sind die erfindungsgemäßen anellierten (Thio)Hydantoine der allgemeinen Formel (I) bzw. (Ia) gegen einige wichtige Unkräuter wesentlich stärker wirksam und zeigen bessere selektive Eigenschaften als beispielsweise 2-(4-Chlor-2-fluor-5-methoxycarbonyl-methoxy-phenyl)-4,5,6,7-tetrahydro-2H-isoindol-1,3-dion, welches ein struktruell ähnlicher vorbekannter Wirkstoff gleicher Wirkungsrichtung ist.

die Erfindung betrifft vorzugsweise Verbindungen der allgemeinen Formel (I), in welcher m für 0, 1 oder 2 steht,

$R^1$ für Wasserstoff, Fluor, Chlor oder Brom steht,

$R^2$ für Wasserstoff, Nitro, Cyano, Fluor, Chlor oder Brom steht oder für einen gegebenenfalls durch Fluor, Chlor, Brom, Cyano, $C_3$-$C_6$-Cycloalkyl (welches gegebenenfalls durch Fluor, Chlor, Brom, Methyl und/oder Ethyl substituiert ist), $C_1$-$C_6$-Alkoxy, $C_1$-$C_4$-Alkoxy-$C_1$-$C_4$-alkoxy, $C_1$-$C_6$-Alkylthio, $C_1$-$C_6$-Alkylsulfinyl, $C_1$-$C_6$-Alkylsulfonyl, $C_1$-$C_4$-Alkylthio-$C_1$-$C_4$-alkoxy, $C_1$-$C_4$-Alkylsulfonyl-$C_1$-$C_4$-alkoxy, $C_3$-$C_6$-Alkenyloxy, $C_3$-$C_6$-Alkinyloxy, $C_3$-$C_6$-Alkenylthio, $C_3$-$C_6$-Alkinylthio, Carboxy, $C_1$-$C_6$-Alkoxy-carbonyl, $C_3$-$C_6$-Alkenyloxy-carbonyl, $C_3$-$C_6$-Alkinyloxy-carbonyl, $C_3$-$C_6$-Cycloalkoxy-carbonyl, $C_3$-$C_6$-Cycloalkenyloxy-carbonyl, $C_3$-$C_6$-Cycloalkyl-$C_1$-$C_4$-alkoxy-carbonyl, $C_3$-$C_6$-Cycloalkenyl-$C_1$-$C_4$-alkoxy-carbonyl, $C_1$-$C_4$-Alkoxy-$C_1$-$C_4$-alkoxy-carbonyl, $C_1$-$C_4$-Alkylthio-$C_1$-$C_4$-alkoxy-carbonyl, Benzyloxy-$C_1$-$C_4$-alkoxy-carbonyl, $C_1$-$C_4$-Alkoxy-carbonyl-$C_1$-$C_4$-alkoxy-carbonyl, $C_1$-$C_4$-Alkylamino-carbonyl-$C_1$-$C_4$-alkoxy-carbonyl, Di-($C_1$-$C_4$-alkyl)-amino-carbonyl-$C_1$-$C_4$-alkoxy-carbonyl, $C_1$-$C_4$-Alkoxy-$C_1$-$C_4$-alkyl-amino-carbonyl, $C_1$-$C_4$-Alkoxy-imino-$C_1$-$C_4$-alkyl, $C_3$-$C_4$-Alkenyloxy-imino-$C_1$-$C_4$-alkyl, $C_1$-$C_4$-Alkoxy-carbonyl-$C_1$-$C_4$-alkoxy-imino-$C_1$-$C_4$-alkyl, $C_1$-$C_6$-Alkylthio-carbonyl, $C_3$-$C_6$-Alkenylthio-carbonyl, $C_3$-$C_6$-Alkinylthio-carbonyl, Carbamoyl, $C_1$-$C_6$-Alkylamino-carbonyl, $C_3$-$C_6$-Alkenylamino-carbonyl, $C_3$-$C_6$-Alkinylamino-carbonyl, Di-($C_1$-$C_4$-alkyl)-amino-carbonyl, Di-($C_3$-$C_4$-alkenyl)-amino-carbonyl und/oder Di-($C_3$-$C_4$-alkinyl)-amino-carbonyl substituierten Rest aus der Reihe $C_1$-$C_8$-Alkyl, $C_1$-$C_6$-Alkoxy, $C_1$-$C_6$-Alkylthio und $C_1$-$C_6$-Alkylamino oder für einen gegebenenfalls durch Fluor und/oder Chlor substituierten Rest aus Reihe $C_3$-$C_6$-Alkenyl, $C_3$-$C_6$-Alkinyl, $C_3$-$C_6$-Alkenyloxy, $C_3$-$C_6$-Alkinyloxy, $C_3$-$C_6$-Alkenylthio, $C_3$-$C_6$-Alkinylthio, $C_1$-$C_6$-Alkylsulfinyl und $C_1$-$C_6$-Alkylsulfonyl steht,

$R^3$ für Wasserstoff, Fluor, Chlor, Brom, Nitro, Cyano oder für einen gegebenenfalls durch Fluor und/oder Chlor substituierten Rest aus der Reihe $C_1$-$C_4$-Alkyl, $C_3$-$C_4$-Alkenyl, $C_3$-$C_4$-Alkinyl, $C_1$-$C_4$-Alkoxy, $C_3$-$C_4$-Alkenyloxy, $C_3$-$C_4$-Alkinyloxy, $C_1$-$C_4$-Alkylthio, $C_3$-$C_4$-Alkenylthio, $C_3$-$C_4$-Alkinylthio, $C_1$-$C_4$-Alkylsulfinyl, $C_1$-$C_4$-Alkylsulfonyl und $C_1$-$C_4$-Alkylamino steht, oder die beiden Reste

$R^2$ und $R^3$ zusammen für die Gruppierung $-X-A-(CO)_n-Y-$steht, worin

n für die Zahlen 0 oder 1 steht,

A für eine direkte Bindung oder für geradkettiges oder verzweigtes und gegebenenfalls durch Fluor und/oder Chlor substituiertes $C_1$-$C_4$-Alkandiyl steht,

X für Sauerstoff oder Schwefel steht und

Y für Sauerstof, Schwefel oder die Gruppierung $N-R^{10}$ steht worin,

$R^{10}$ für Wasserstoff oder gegebenenfalls durch Fluor und/oder Chlor substituierte Reste aus der Reihe $C_1$-$C_4$-Alkyl, $C_3$-$C_4$-Alkenyl und $C_3$-$C_4$-Alkinyl steht,

in welcher weiter

$R^4$ für Wasserstoff, Fluor, Chlor oder Brom steht,

$R^5$ für Wasserstoff, Fluor, Chlor oder Brom steht,

$R^6$ für Wasserstoff, gegebenenfalls durch Fluor und/oder Chlor substituiertes $C_1$-$C_6$-Alkyl oder gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_2$-Fluoralkoxy substituiertes Phenyl oder Naphthyl steht,

$R^7$ für Wasserstoff oder $C_1$-$C_4$-Alkyl steht,

$R^8$ für Wasserstoff oder $C_1$-$C_4$-Alkyl steht,

$R^9$ für Wasserstoff oder $C_1$-$C_4$-Alkyl steht und

Q für Sauerstoff oder Schwefel steht,

-mit der Maßgabe, daß

1. wenigstens einer der Reste $R^1$ bis $R^9$ von Wasserstoff verschieden ist,

2. $R^3$ nur dann für Methyl, Methoxy oder Chlor steht, wenn wenigstens einer der Reste $R^1$, $R^2$, $R^4$ und $R^5$ von Wasserstoff verschieden ist und

3. die Reste $R^2$ und $R^4$ nur dann gleichzeitig für Chlor stehen, wenn wenigstens einer der Reste $R^1$, $R^3$ und $R^5$ von Wasserstoff verschieden ist.

Die Erfindung betrifft insbesondere Verbindungen der allgemeinen Formel (I), in welcher m für Null steht,

$R^1$ für Wasserstoff steht,

$R^2$ für Wasserstoff, Nitro, Cyano, Fluor, Chlor oder Brom oder für einen gegebenenfalls durch Fluor, Chlor, Cyclopropyl (welches durch Chlor und/oder Methyl substituiert sein kann), $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, Carboxy, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl, Di-($C_1$-$C_2$-alkyl)-aminocarbonyl, $C_1$-$C_2$-Alkoxy-$C_1$-$C_2$-alkyl-amino-carbonyl, $C_3$-$C_4$-Alkenyloxy-imino-$C_1$-$C_2$-alkyl, $C_1$-$C_2$-Alkoxy-imino-$C_1$-$C_2$-alkyl, $C_1$-$C_2$-Alkoxy-carbonyl-$C_1$-$C_2$-alkoxy-imino-$C_1$-$C_2$-alkyl substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Methyl oder Trifluormethyl steht, oder die beiden Reste

$R^2$ und $R^3$ zusammen für die Gruppierung -X-A-$(CO)_n$-Y-stehen, worin

n für die Zahlen 0 oder 1 steht,

A für eine direkte Bindung oder für Methylen, Ethylen, Propylen oder Butylen steht,

X für Sauerstoff steht,

Y für Sauerstoff oder die Gruppierung N-$R^{10}$ steht, worin

$R^{10}$ für Wasserstoff, $C_1$-$C_4$-Alkyl, $C_3$-$C_4$-Alkenyl oder $C_3$-$C_4$-Alkinyl steht,

in welcher weiter

$R^4$ für Wasserstoff steht,

$R^5$ für Wasserstoff, Fluor oder Chlor steht,

$R^6$ für Wasserstoff, $C_1$-$C_4$-Alkyl oder für gegebenenfalls durch $C_1$-$C_2$-Alkoxy oder Nitro substituiertes Phenyl steht sowie

$R^7$, $R^8$ und $R^9$ unabhängig voneinander für Wasserstoff, Methyl oder Ethyl stehen und

Q für Sauerstoff oder Schwefel steht,

-mit der Maßgabe, daß

1. wenigstens einer der Reste $R^2$, $R^3$, $R^5$, $R^6$, $R^7$, $R^8$ und $R^9$ von Wasserstoff verschieden ist und

2. $R^3$ nur dann für Chlor steht, wenn wenigstens einer der Reste $R^2$ und $R^5$ von Wasserstoff verschieden ist.

Die Erfindung betrifft ganz besonders bevorzugt Verbindungen der Formel (I), in welcher m für Null steht,

$R^1$ für Wasserstoff steht,

$R^2$ für Wasserstoff, Fluor, Chlor oder Brom oder für einen gegebenenfalls durch Fluor, Chlor, Cyclopropyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Methyl oder Trifluormethyl steht, oder die beiden Reste

$R^2$ und $R^3$ zusammen für die Gruppierung -X-A-$(CO)_n$-Y-stehen, worin

n für die Zahlen 0 oder 1 steht,

A für Methylen, Ethylen, Propylen oder Butylen steht,

X für Sauerstoff steht,

Y für Sauerstoff oder die Gruppierung N-$R^{10}$ steht, worin

$R^{10}$ für Wasserstoff, $C_1$-$C_4$-Alkyl, $C_3$-$C_4$-Alkenyl oder $C_3$-$C_4$-Alkinyl steht,

in welcher weiter

$R^4$ für Wasserstoff steht,

$R^5$ für Wasserstoff oder Fluor steht,

$R^6$, $R^7$, $R^8$ und $R^9$ für Wasserstoff stehen und
Q für Sauerstoff oder Schwefel steht,
-mit der Maßgabe, daß

1. wenigstens einer der Reste $R^2$, $R^3$ und $R^5$ von Wasserstoff verschieden ist und
2. $R^3$ nur dann für Chlor steht, wenn wenigstens einer der Reste $R^2$ und $R^5$ von Wasserstoff verschieden ist.

Die Substituenten $R^1$, $R^2$, $R^3$, $R^4$, $R^5$, $R^6$, $R^7$, $R^8$, $R^9$, Q und m der Formel (Ia) besitzen bevorzugt bzw. besonders bevorzugt bzw. ganz besonders bevorzugt diejenige Bedeutung, die bei der Beschreibung der Verbindungen der Formel (I) für diese Reste als bevorzugt bzw. besonders bevorzugt bzw. ganz besonders bevorzugt genannt wurden.

Verwendet man für das erfindungsgemäße Verfahren beispielsweise Thiazolidin-4-carbonsäure-methylester und 4-Fluor-phenylisothiocyanat als Ausgangsstoffe, so kann der Verlauf des erfindungsgemäßen Verfahrens durch das folgende Formelschema wiedergegeben werden:

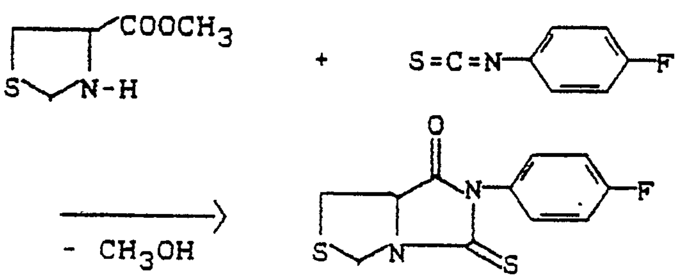


Die bei dem erfindungemäßen Verfahren zur Herstellung von Verbindungen der Formel (I) als Ausgangsstoffe benötigten Aryliso(thio)cyanate sind durch die Formel (II) allgemein definiert.

In Formel (II) haben $R^1$, $R^2$, $R^3$, $R^4$, $R^5$ und Q vorzugsweise bzw. insbesondere die gleichen Bedeutungen, wie sie bereits im Zusammenhang mit der Beschreibung der erfindungsgemäßen Verbindungen der Formel (I) vorzugsweise bzw. als insbesondere bevorzugt angegeben sind.

Als Beispiele für die Ausgangsstoffe der Formel (II) seien genannt:
2-Fluor-, 3-Fluor-, 4-Fluor-und 2,4-Difluor-phenylisocyanat bzw. -phenylisothiocyanat, 2-Chlor-, 3-Chlor-, 4-chlor-, 2,3-Dichlor-, 2,4-Dichlor und 3,4-Dichlorphenylisocyanat bzw. -phenylisothiocyanat, 3-Brom-und 4-Brom-phenylisocyanat bzw. -phenylisothiocyanat, 3-Trifluormethyl-und 4-Trifluromethyl-phenylisocyanat bzw. -phenylisothiocyanat, 3-Chlor-4-trifluromethyl-und 4-Chlor-3-trifluormethyl-phenylisocyanat bzw. -phenylisothiocyanat, 3-Chlor-4-methyl-und 4-Chlor-3-methyl-phenylisocyanat bzw. -phenylisothiocyanat, 3-Methoxy-, 3-Ethoxy-, 4-Methoxy, 4-Ethoxy-und 3-Isopropoxy-phenylisocyanat bzw. -phenylisothiocyanat, 4-Nitro-phenylisocyanat und 4-Nitro-phenylisothiocyanat, 2,4-Dichlor-5-methoxy-, 2,4-Dichlor-5-ethoxy-und 2,4-Dichlor-5-isopropoxy-phenylisocyanat bzw. -phenylisothiocyanat, 2-Fluor-4-chlor-5-methoxycarbonylmethoxy-phenylisocyanat bzw. -phenylisothiocyanat, 2,4-Dichlor-5-methoxycarbonylmethoxy-phenylisocyanat bzw. -phenylisothiocyanat, 3-Methoxy-4-chlor-phenylisocyanat bzw. -phenylisothiocyanat, 3-Isopropoxy-4-chlor-phenylisocyanat bzw. -phenylisothiocyanat, 3-(1-Methoxycarbonyl)-ethoxy-4-chlorphenylisocyanat bzw. -phenylisothiocyanat, 2,4-Dichlor-5-(1-methoxycarbonyl)-ethoxy-phenylisocyanat bzw. -phenylisothiocyanat, 3-Allyloxy-4-chlor-phenylisocyanat bzw. -phenylisothiocanat, 2-Fluor-4-chlor-5-allyloxy-phenyl-isocyanat bzw. -phenylisothiocyanat, 2,4-Dichlor-5-allyloxy-phenylisocyanat bzw. -phenylisothiocyanat, 3-Propargyloxy-4-chlor-phenylisocyanat bzw. -phenylisothiocyanat, 2,4-Dichlor-5-propargyloxy-phenylisocyanat bzw. -phenylisothiocyanat, 2-Fluor-4-chlor-5-propargyloxy-phenylisocyanat bzw. -phenylisothiocyanat.

Die Ausgangsstoffe der Formel (II) sind teilweise bekannt und können nach an sich bekannten Verfahren hergestellt werden (vgl. EP-A 70 389, EP-A 69 855, EP-A 75 267).

Neu sind Ausgangsstoffe der Formel (IIa)

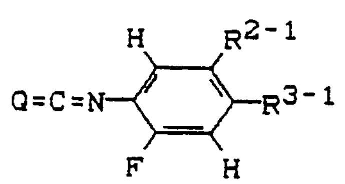

(IIa)

in welcher

Q für Sauerstoff oder Schwefel steht,

$R^{2-1}$ für einen gegebenenfalls substituierten rest aus der Reihe Alkoxy, Alkenyloxy, Alkinyloxy, Alkylthio, Alkenylthio oder Alkinylthio steht und

$R^{3-1}$ für Halogen oder für gegebenenfalls substituiertes Alkyl steht

- mit Ausnahme der Verbindungen, bei denen $R^{2-1}$ für Propoxy, Allyloxy, Propargyyloxy oder Acetylmethoxy steht und gleichzeitig $R^{3-1}$ für Chlor steht.

Bevorzugt sind diejenigen Verbindungen der Formel (IIa), in denen

Q für Sauerstoff oder Schwefel steht,

$R^{2-1}$ für einen gegebenenfalls durch Fluor, Chlor, Brom, Cyano, $C_3$-$C_6$-Cycloalkyl (welches gegebenenfalls durch Fluor, Chlor, Brom, Methyl und/oder Ethyl substituiert ist), $C_1$-$C_6$-Alkoxy, $C_1$-$C_4$-Alkoxy-$C_1$-$C_4$-alkoxy, $C_1$-$C_6$-Alkylthio, $C_1$-$C_6$-Alkylsulfinyl, $C_1$-$C_6$-Alkylsulfonyl, $C_1$-$C_4$-Alkylthio-$C_1$-$C_4$-alkoxy, $C_1$-$C_4$-Alkylsulfonyl-$C_1$-$C_4$-alkoxy, $C_3$-$C_6$-Alkenyloxy, $C_3$-$C_6$-Alkinyloxy, $C_3$-$C_6$-Alkenylthio, $C_3$-$C_6$-Alkinylthio, Carboxy, $C_1$-$C_6$-Alkoxy-carbonyl, $C_3$-$C_6$-Alkenyloxy-carbonyl, $C_3$-$C_6$-Alkinyloxy-carbonyl, $C_3$-$C_6$-Cycloalkyloxy-carbonyl, $C_3$-$C_6$-Cycloalkenyloxy-carbonyl, $C_3$-$C_6$-Cycloalkyl-$C_1$-$C_4$-alkoxy-carbonyl, $C_3$-$C_6$-Cycloalkenyl-$C_1$-$C_4$-alkoxy-carbonyl, $C_1$-$C_4$-Alkoxy-$C_1$-$C_4$-alkoxy-carbonyl, $C_1$-$C_4$-Alkylthio-$C_1$-$C_4$-alkoxy-carbonyl, Benzyloxy-$C_1$-$C_4$-alkoxy-carbonyl, $C_1$-$C_4$-Alkoxy-carbonyl-$C_1$-$C_4$-alkoxy-carbonyl, $C_1$-$C_4$-Alkylamino-carbonyl-$C_1$-$C_4$-alkoxy-carbonyl, Di-($C_1$-$C_4$-alkyl)-amino-carbonyl-$C_1$-$C_4$-alkoxy-carbonyl, $C_1$-$C_4$-Alkoxy-$C_1$-$C_4$-alkyl-amino-carbonyl, $C_1$-$C_4$-Alkoxy-imino-$C_1$-$C_4$-alkyl, $C_3$-$C_4$-Alkenyloxy-imino-$C_1$-$C_4$-alkyl, $C_1$-$C_4$-Alkoxy-carbonyl-$C_1$-$C_4$-alkoxy-imino-$C_1$-$C_4$-alkyl, $C_1$-$C_6$-Alkylthio-carbonyl, $C_3$-$C_6$-Alkenylthio-carbonyl, $C_3$-$C_6$-Alkinylthio-carbonyl, Carbamoyl, $C_1$-$C_6$-Alkylamino-carbonyl, $C_3$-$C_6$-Alkenylamino-carbonyl, $C_3$-$C_6$-Alkinylamino-carbonyl, Di-($C_1$-$C_4$-alkyl)-amino-carbonyl, Di-($C_3$-$C_4$-alkenyl)-amino-carbonyl und/oder Di-($C_3$-$C_4$-alkinyl)-aminocarbonyl substituierten Rest aus der Reihe $C_1$-$C_6$-Alkoxy und $C_1$-$C_6$-Alkylthio oder für einen gegebenenfalls durch Fluor und/oder Chlor substituierten Rest aus der Reihe $C_3$-$C_6$-Alkenyloxy, $C_3$-$C_6$-Alkinyloxy, $C_3$-$C_6$-Alkenylthio oder $C_3$-$C_6$-Alkinylthio steht und

$R^{3-1}$ für Fluor, Chlor, Brom oder für gegebenenfalls durch Fluor und/oder Chlor substituiertes $C_1$-$C_4$-Alkyl steht,

-mit Ausnahme der Verbindungen, bei denen $R^{2-1}$ für Propoxy, Allyloxy, Propargyloxy oder Acetylmethoxy steht und gleichzeitig $R^{3-1}$ für Chlor steht.

Besonders bevorzugt sind die Verbindungen der Formel (IIa), in denen

Q für Sauerstoff oder Schwefel steht,

$R^{2-1}$ für einen gegebenenfalls durch Fluor, Chlor, Cyclopropyl (welches durch Chlor und/oder Methyl substituiert sein kann), $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, Carboxy, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloakyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl, Di-($C_1$-$C_2$-alkyl)-aminocarbonyl, $C_1$-$C_2$-Alkoxy-$C_1$-$C_2$-alkyl-amino-carbonyl, $C_3$-$C_4$-Alkenyloxy-imino-$C_1$-$C_2$-alkyl, $C_1$-$C_2$-Alkoxy-imino-$C_1$-$C_2$-alkyl, $C_1$-$C_2$-Alkoxy-carbonyl-$C_1$-$C_2$-alkoxy-imino-$C_1$-$C_2$-alkyl substituierten Rest aus der Reihe $C_1$-$C_4$-Alkoxy und $C_1$-$C_4$-Alkylthio oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht und

$R^{3-1}$ für Fluor, Chlor, Brom, Methyl oder Trifluormethyl steht,

-mit Ausnahme der Verbindungen, bei denen $R^{2-1}$ für Propoxy, Allyloxy, Propargyloxy oder Acetylmethoxy steht und gleichzeitig $R^{3-1}$ für Chlor steht.

Ganx besonders bevorzugt sind die Verbindungen der Formel (IIa), in denen Q für Sauerstoff oder Schwefel steht,

$R^{2-1}$ für einen gegebenenfalls durch Fluor, Chlor, Cyclopropyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyl-oxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl substituierten Rest aus der Reihe $C_1$-$C_4$-Alkoxy und $C_1$-$C_4$-Alkylthio oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht und

$R^{3-1}$ für Fluor, Chlor, Brom, Methyl oder Trifluormethyl steht

-mit Ausnahme der Verbindungen, bei denen $R^{2-1}$ für Propoxy, Allyloxy, Propargyloxy oder Acetylme-

thoxy steht und gleichzeitig $R^{3-1}$ für Chlor steht.

Man erhält die neuen Aryliso(thio)cyanate der allgemeinen Formel (IIa), wenn man Arylamine der allgemeinen Formel (IV)

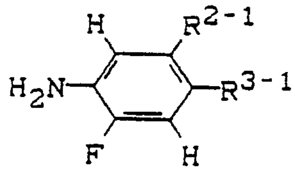

(IV)

in welcher

$R^{2-1}$ und $R^{3-1}$ die oben angegebenen Bedeutungen haben,

mit Phosgen bzw. mit Thiophosgen, gegebenenfalls in Gegenwart eines Verdünnungsmittels, wie z. B. Toluol bzw. Chloroform und Wasser, bei Temperaturen zwischen -10 °C und +120 °C umsetzt.

In entsprechnder Weise lassen sich auch die bekannten Verbindungen der Formel (II) herstellen.

Die Arylamine der allgemeinen Formel (IV) sind bekannt und/oder können nach an sich bekannten Verfahren hergestellt werden (vgl. EP-A 61 741, EP-A 69 855, EP-A 70 389, EP-A 75 267, EP-A 83 055, EP-A 95 192, EP-A 211 805).

Die bei erfindungsgemäßen Verfahren weiter als Ausgangsstoffe benötigen Thiazolidincarbonsäure-(ester sind durch die Formel (III) allgemein definiert.

In Formel (III) haben m, $R^6$, $R^7$, $R^8$, und $R^9$ vorzugsweise bzw. insbesondere die gleichen Bedeutungen, wie sie bereits im Zusmamenhang mit der Beschreibung der erfindungsgemäßen Verbindungen der Formel (I) vorzugsweise bzw. als insbesondere bevorzugt angegeben sind und R steht vorzugsweise für Wasserstoff oder $C_1$-$C_4$-Alkyl, insbesondere für Methyl oder Ethyl.

Als Beispiele für die Ausgangsstoffe der Formel (III) seien genannt:

Thiazolidin-4-carbonsäure sowie deren Methylester und Ethylester, 2-Methyl-, 2-Ethyl-, 2-Propyl-, 2-Isopropyl-, 2-Phenyl-, 2-(4-Nitro-phenyl)-, 2-(4-Methoxy-phenyl)-, 2,2-Dimethyl-, 5-Methyl-und 5,5-Dimethyl-thiazolidin-4-carbonsäure sowie deren Methylester und Ethylester, ferner die entpreschenden S-Oxide und S,S-Dioxide.

Die Ausgangsstoffe der Formel (III) sind bekannt und/oder können nach an sich bekannten Verfahren hergestelltwerden (vgl. z. B. J. Am. Chem. Soc. 79 (1957), 1650 - 1651; Tetrahedron 29 (1973), 3389 -3398).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und-ethylester, Nitrile z. B. Acetonitril und Propionitril, Amide wie z. B. Dimethylformamid, Di-methylacetamid und N-Methyl-pyrrolidon sowie Dimethylsulfoxid, Tetramethylensulfon und Hexamethylphosphorsäuretriamid.

Als Säureakzeptoren können beidem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C vorzugsweise bei Temperaturen zwischen 10 °C und 80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die Ausgangsstoffe der allgemeinen Formeln (II) und (III) im allgemeinen in angenähert äquimolaren Mengen eingesetzt. Es ist jedoch auch

möglich, eine der beiden jeweils eingesetzten Komponenten in einem mehr oder weniger großen Überschuß zu verwenden. Die Umsetzungen werden im allgemeinen in einem geeigneten Verdünnungsmittel, gegebenenfalls in Gegenwart eines Säureakzeptors durchgeführt und das Reaktionsgemisch wird bis zum Ende der Umsetzung bei der jeweils erforderlichen Temperatur gerührt.

Die Aufarbeitung erfolgt beim erfindunggemäßen Verfahren nach üblichen Methoden. Beispielsweise wird das Reaktionsgemisch, gegebenenfalls nach Einengen, mit verdünnter Salzsäure und einem mit Wasser praktisch nicht mischbaren organischen Lösungsmittel, wie z. B. Toluol, geschüttelt, die organische Phase abgetrennt, mit Wasser gewaschen, getrocknet und filtriert. Der nach Einengen des Filtrats erhaltene Rückstand enthält im wesentlichen das Produkt der Formel (I), welches gegebenenfalls durch Anreiben mit einem organischen Lösungsmittel zur Kristallisation gebracht wird.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eignen sich insbesondere zur selektiven Bekämpfung dikotyler Unkräuter in monokotylen Kulturen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder -

schaumzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoff, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 un d95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethyl-harnstoff zur Unkrautbekämpfung in Getriede; 4-Amino-3-methyl-6-phenyl-1,2,4-triazine-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage; ferner auch Atrazin, Bentazon, Bifenox, Bromoxynil, Chlorsulfuron, Chlortoluron, Cyanazin, Fluoroxypyr, Illoxan, Imazamethabenz, Ioxynil, Isoproturon, MCPA, MCPP, Metsulfuron-Methyl, Pendimethalin, Pyridate, Terbutryne, Fenoxaprop, Amethydione, 2,4-D, 2,4-DP, [(4-Amino,3,5-dichlor-6-fluor-2-pyridinyl)-oxy]-essigsäure und deren 1-Methyl-heptylester und 2-[4-(3,5-Dichlor-2-pyridyl-oxy)-phenoxy]-propionsäure-(trimethylsilylmethylester). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

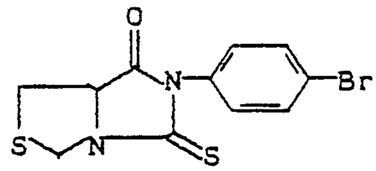

Eine Mischung aus 1,61 g (0,01 Mol) Thiazolidin-4-carbonsäure-ethylester, 2,0 g (0,009 Mol) 4-Bromphenylisothiocyanat und 50 ml Toluol wird 60 Minuten bei 20 °C gerührt, dann mit 10-%iger Salzsäure (3 mal 10 ml) und Wasser (3 mal 10 ml) gewaschen, über Natriumsulfat getrocknet und filtriert. Nach Einengen des Filtrats wird der Rückstand durch Verreiben mit n-Pentan zur Kristallisation gebracht.

Man erhält 2,6 g (88 % der Theorie) 5-(4-Brom-phenyl)-perhydroimidazo[1,5-c]thiazol-6-on-4-thion vom Schmelzpunkt 171 °C.

Analog Beispiel 1 und gemäß der allgemeinen Beschreibung des erfindungsgemäßen Verfahrens können die in der nachstehenden Tabelle 1 aufgeführten Verbindungen der allgemeinen Formel (I) hergestellt werden:

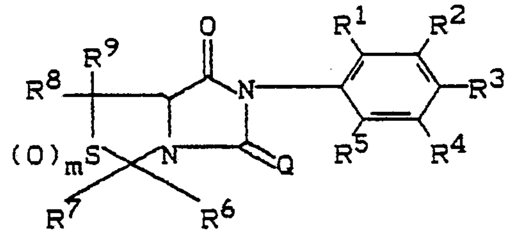

(I)

Tabelle 1: Beispiele für die Verbindungen der Formel (I)

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 2 | H | H | Cl | H | H | H | H | H | H | O | 0 | 125 |
| 3 | H | Cl | H | H | H | H | H | H | H | O | 0 | 74 |
| 4 | H | Cl | Cl | H | H | H | H | H | H | O | 0 | 105 |
| 5 | H | Cl | $CF_3$ | H | H | H | H | H | H | O | 0 | 92 |
| 6 | H | $CF_3$ | H | H | H | H | H | H | H | O | 0 | 97 |
| 7 | H | H | $OC_2H_5$ | H | H | H | H | H | H | O | 0 | 98 |
| 8 | H | Cl | $CH_3$ | H | H | H | H | H | H | O | 0 | 122 |
| 9 | H | H | H | H | F | H | H | H | H | O | 0 | 112 |
| 10 | H | H | F | H | H | H | H | H | H | O | 0 | 126 |
| 11 | H | H | H | H | Cl | H | H | H | H | O | 0 | 114 |
| 12 | H | F | H | H | H | H | H | H | H | O | 0 | 54 |
| 13 | H | H | Cl | H | H | H | H | H | H | S | 0 | 146 |
| 14 | H | H | F | H | H | H | H | H | H | S | 0 | 170 |
| 15 | H | $OCH_3$ | Cl | H | F | H | H | H | H | O | 0 | 135 |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 16 | H | H | Cl | H | H | 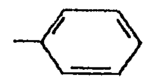 | H | H | H | O | 0 | 153 |
| 17 | H | H | $OC_2H_5$ | H | H | $CH_3$ | H | H | H | O | 0 | 110 |
| 18 | H | H | Cl | H | H | $CH_3$ | H | H | H | S | 0 | 161 |
| 19 | H | H | F | H | H | $CH_3$ | H | H | H | S | 0 | 139 |
| 20 | H | H | H | H | H | H | $CH_3$ | H | H | S | 0 | 143 |
| 21 | H | Cl | H | H | H | 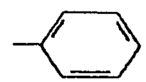 | H | H | H | S | 0 | 140 |
| 22 | H | H | H | H | H | 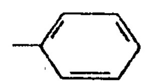 | H | H | H | S | 0 | 168 |
| 23 | H | $CF_3$ | H | H | H | 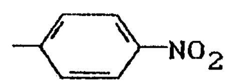 | H | H | H | S | 0 | 131 |
| 24 | H | H | Br | H | H | 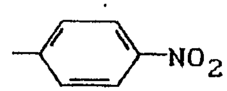 | H | H | H | S | 0 | 148 |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 25 | H | H | Br | H | H | 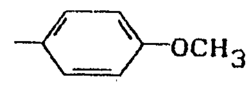 | H | H | H | O | 0 | 98 |
| 26 | H | H | Br | H | H | 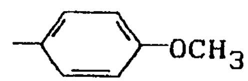 | H | H | H | S | 0 | 116 |
| 27 | H | H | Cl | H | H | $CH(CH_3)_2$ | H | H | H | O | 0 | 144 |
| 28 | H | H | H | H | F | $CH(CH_3)_2$ | H | H | H | S | 0 | 90 |
| 29 | H | Cl | H | H | H | $CH(CH_3)_2$ | H | H | H | S | 0 | 90 |
| 30 | H | H | Br | H | H | $CH(CH_3)_2$ | H | H | H | S | 0 | 93 |
| 31 | H | H | Cl | H | H | 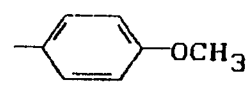 | H | H | H | O | 0 | (amorph) |
| 32 | H | H | $NO_2$ | H | H | H | H | H | H | O | 0 | 98 |
| 33 | H | $OCH_3$ | H | H | H | H | H | H | H | O | 0 | (amorph) |
| 34 | H | H | Br | H | H | $CH_3$ | H | $CH_3$ | $CH_3$ | S | 0 | 159 |
| 35 | H | H | Cl | H | H | $CH_3$ | H | $CH_3$ | $CH_3$ | O | 0 | 116 |
| 36 | H | H | Cl | H | H | $CH_3$ | H | $CH_3$ | $CH_3$ | S | 0 | 151 |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 37 | H | H | Br | H | H | H | H | $CH_3$ | $CH_3$ | S | 0 | 128 |
| 38 | H | H | Cl | H | H | H | H | $CH_3$ | $CH_3$ | S | 0 | 119 |
| 39 | H | H | Cl | H | Cl | H | H | H | H | S | 0 | 136 |
| 40 | H | $OCH_3$ | Cl | H | Cl | H | H | H | H | S | 0 | 163 |
| 41 | H | $OCH(CH_3)_2$ | Cl | H | Cl | H | H | H | H | S | 0 | 153 |
| 42 | H | $OCH_2COOCH_3$ | Cl | H | Cl | H | H | H | H | S | 0 | 97 |
| 43 | H | $OCH_3$ | Cl | H | H | H | H | H | H | S | 0 | 170 |
| 44 | H | $OCH(CH_3)_2$ | Cl | H | H | H | H | H | H | S | 0 | 119 |
| 45 | H | $OCH_2COOCH_3$ | Cl | H | H | H | H | H | H | S | 0 | 151 |
| 46 | H | $OCH(CH_3)COOCH_3$ | Cl | H | Cl | H | H | H | H | S | 0 | 175 |
| 47 | H | $OCH(CH_3)COOCH_3$ | Cl | H | H | H | H | H | H | S | 0 | 90 |
| 48 | H | $OCH_2CH=CH_2$ | Cl | H | H | H | H | H | H | S | 0 | 139 |
| 49 | H | $-NH-CO-CH_2-O-$ | | H | F | H | H | H | H | S | 0 | 216 |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/° C |
|---|---|---|---|---|---|---|---|---|---|---|---|---|
| 50 | H | Cl | F | Cl | F | H | H | H | H | O | 0 | 137 |
| 51 | H | $OCH_2CH=CH_2$ | Cl | H | Cl | H | H | H | H | S | 0 | 127 |
| 52 | H | $OCH_2C\equiv CH$ | Cl | H | Cl | H | H | H | H | S | 0 | 161 |
| 53 | H | $OCH_2C\equiv CH$ | Cl | H | H | H | H | H | H | S | 0 | 136 |
| 54 | H | $OCH_2C\equiv CH$ | Cl | H | F | H | H | H | H | S | 0 | 134 |
| 55 | H | $OCHCOOCH_3$<br>\|<br>$CH_3$ | Cl | H | F | H | H | H | H | S | 0 | 86 |
| 56 | H | $OCH_3$ | Cl | H | F | H | H | H | H | S | 0 | 187 |
| 57 | H | $OCH_2COOCH_3$ | Cl | H | F | H | H | H | H | S | 0 | 114 |
| 58 | H | $OCH(CH_3)_2$ | Cl | H | F | H | H | H | H | S | 0 | 120 |
| 59 | H | $OCH(CH_3)_2$ | Br | H | F | H | H | H | H | S | 0 | |
| 60 | H | $OC_2H_5$ | Cl | H | F | H | H | H | H | S | 0 | |
| 61 | H | $OC_2H_5$ | Br | H | F | H | H | H | H | S | 0 | |
| 62 | H | $OCH_2CH_2CH_3$ | Cl | H | F | H | H | H | H | O | 0 | |
| 63 | H | $OCH_2CH(CH_3)_2$ | Cl | H | F | H | H | H | H | O | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 64 | H | $OCHCH_2CH_3$<br>\| <br>$CH_3$ | Cl | H | F | H | H | H | H | O | 0 | |
| 65 | H | $OCH_2CH{=}CH_2$ | Cl | H | F | H | H | H | H | S | 0 | 127 |
| 66 | H | $OCHCH{=}CH_2$<br>\| <br>$CH_3$ | Cl | H | F | H | H | H | H | S | 0 | |
| 67 | H | $OCH_2CH{=}CH_2$ | Br | H | F | H | H | H | H | O | 0 | |
| 68 | H | $OCH_2C{\equiv}CH$ | Br | H | F | H | H | H | H | S | 0 | |
| 69 | H | $OCH(CH_3)_2$ | Cl | H | F | H | H | H | H | O | 1 | |
| 70 | H | $OCH_2COOCH_3$ | Cl | H | F | H | H | H | H | O | 2 | |
| 71 | H | $OCH_2COOC_2H_5$ | Cl | H | F | H | H | H | H | S | 0 | |
| 72 | H | $OCH_2COOCH(CH_3)_2$ | Cl | H | F | H | H | H | H | S | 0 | |
| 73 | H | $OCH_2COO$ 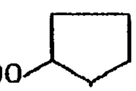 | Cl | H | F | H | H | H | H | S | 0 | |
| 74 | H | $SCH_3$ | Cl | H | F | H | H | H | H | O | 0 | |
| 75 | H | $OCH_2COOC_5H_{11}$ | Cl | H | F | H | H | H | H | S | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 76 | H | $OCH_2COOCH_2$- 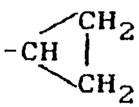 | Cl | H | F | H | H | H | H | O | 0 | |
| 77 | H | $OCH_2COOCH_2$ 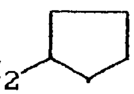 | Cl | H | F | H | H | H | H | O | 0 | |
| 78 | H | $OCH_2CH_2OC_2H_5$ | Cl | H | F | H | H | H | H | O | 0 | |
| 79 | H | $OCH_2CH_2SC_2H_5$ | Cl | H | F | H | H | H | H | S | 0 | |
| 80 | H | $SCH_2CH_2SCH_3$ | Cl | H | F | H | H | H | H | S | 0 | |
| 81 | H | $SCH_2COOCH_2$ 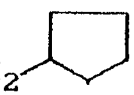 | Cl | H | F | H | H | H | H | S | 0 | |
| 82 | H | $OCH_2$-$COOCH_2$ 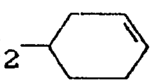 | Cl | H | F | H | H | H | H | O | 0 | |
| 83 | H | $OCH_2COOCH_2CH{=}CH_2$ | Cl | H | F | H | H | H | H | S | 0 | |
| 84 | H | $OCH_2COOCH_2C{\equiv}CH$ | Cl | H | F | H | H | H | H | S | 0 | |
| 85 | H | $SC_2H_5$ | Cl | H | H | H | H | H | H | O | 0 | |
| 86 | H | $SCH(CH_3)_2$ | Cl | H | F | H | H | H | H | S | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/° C |
|---|---|---|---|---|---|---|---|---|---|---|---|---|
| 87 | H | $SCH_2CH=CH_2$ | Cl | H | Cl | H | H | H | H | S | 0 | |
| 88 | H | $SCH_2C \equiv CH$ | Cl | H | Cl | H | H | H | H | O | 0 | |
| 89 | H | $SCH_2COOC_5H_{11}$ | Cl | H | F | H | H | H | H | S | 0 | |
| 90 | H | $SCH_2COO$ 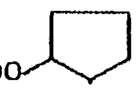 | Cl | H | F | H | H | H | H | S | 0 | |
| 91 | H | $OCH_2CON(CH_3)_2$ | Cl | H | F | H | H | H | H | S | 0 | |
| 92 | H | $CH_3$<br>$OCH_2CON$<br>$OCH_3$ | Cl | H | F | H | H | H | H | O | 0 | |
| 93 | H | $OC=N-OCH_3$<br>$CH_3$ | Cl | H | F | H | H | H | H | S | 0 | |
| 94 | H | $OCH_2CH=N-OCH_3$ | Cl | H | F | H | H | H | H | S | 0 | |
| 95 | H | $OCHCH=N-OC_2H_5$<br>$CH_3$ | Cl | H | H | H | H | H | H | S | 0 | |
| 96 | H | $OCH_2CH=N-OCH_2CH=CH_2$ | Cl | H | F | H | H | H | H | S | 0 | |
| 97 | H | $OCH_2CH=N-OCH_2COOCH_3$ | Cl | H | F | H | H | H | H | S | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelz-punkt/°C |
|---|---|---|---|---|---|---|---|---|---|---|---|---|
| 98 | H | $SCH_2COOCH_2CH=CH_2$ | Cl | H | F | H | H | H | H | S | 0 | |
| 99 | H | $SCH_2COOCH_2C\equiv CH$ | Cl | H | F | H | H | H | H | S | 0 | |
| 100 | H | $OCH_2COOCH_2COOCH_3$ | Cl | H | F | H | H | H | H | S | 0 | |
| 101 | H | 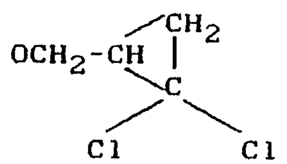 | Cl | H | H | H | H | H | H | O | 0 | |
| 102 | H | 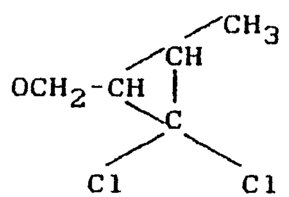 | Cl | H | F | H | H | H | H | O | 0 | |
| 103 | H | $OCHF_2$ | Cl | H | F | H | H | H | H | S | 0 | |
| 104 | H | $OCF_2CHFCl$ | Cl | H | F | H | H | H | H | S | 0 | |
| 105 | H | 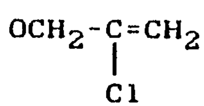 | Cl | H | F | H | H | H | H | S | 0 | |
| 106 | H | $OCF_2CHF_2$ | Cl | H | H | H | H | H | H | O | 0 | |
| 107 | H | $OCH_2CH=CHCl$ | Cl | H | F | H | H | H | H | O | 0 | |

<u>Tabelle 1</u> - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 108 | H | $OCClF_2$ | Cl | H | Cl | H | H | H | H | O | 0 | |
| 109 | H | $-N-CO-CH_2-O-$<br>\|<br>$CH_2C{\equiv}CH$ | | H | F | H | H | H | H | S | 0 | |
| 110 | H | $-N-CO-CH_2-O-$<br>\|<br>$CH_3$ | | H | Cl | H | H | H | H | S | 0 | |
| 111 | H | $-OCH_2-C=N-OCH_3$<br>\|<br>$COOC_2H_5$ | Cl | H | F | H | H | H | H | S | 0 | |
| 112 | H | $-OCH_2CO-NHCH(CH_3)_2$ | Cl | H | F | H | H | H | H | S | 0 | |
| 113 | H | $-N-CO-CH_2-O-$<br>\|<br>$CH_2CH=CH_2$ | | H | F | H | H | H | H | S | 0 | |
| 114 | H | $-SCH_2CH=CH_2$ | Cl | H | H | H | H | H | H | S | 0 | 99 |
| 115 | H | $-SCH_2COOCH_3$ | Cl | H | H | H | H | H | H | S | 0 | |
| 116 | H | $-SCH_2COOCH_3$ | Cl | H | H | H | H | H | H | O | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 117 | H | $-OCH_2COOH$ | Cl | H | H | H | H | H | H | S | 0 | 187 |
| 118 | H | $-OCH_2CH=CH_2$ | Cl | H | H | H | H | H | H | O | 0 | 75 |
| 119 | H | H | Cl | H | F | H | H | H | H | S | 0 | 135 |
| 120 | H | H | Cl | H | F | H | H | H | H | O | 0 | 97 |
| 121 | H | $-O-CHCOOCH_3$<br>\|<br>$CH_3$ | $CH_3$ | H | H | H | H | H | H | O | 0 | |
| 122 | H | $-O-CHCOOCH_3$<br>\|<br>$CH_3$ | $CH_3$ | H | H | H | H | H | H | S | 0 | 82 |
| 123 | H | $-OCH_2CH=CH_2$ | $CH_3$ | H | H | H | H | H | H | O | 0 | 99 |
| 124 | H | $-OCH_2CH=CH_2$ | $CH_3$ | H | H | H | H | H | H | S | 0 | 165 |
| 125 | H | $-OCH_2C\equiv CH$ | $CH_3$ | H | H | H | H | H | H | O | 0 | 104 |
| 126 | H | $-OCH(CH_3)_2$ | Cl | H | F | H | H | H | H | O | 0 | |
| 127 | H | $-OCH_2C\equiv CH$ | Cl | H | F | H | H | H | H | O | 0 | |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 128 | H | $-OCH_2CH=CH_2$ | Cl | H | F | H | H | H | H | O | O | |
| 129 | H | $-OCH_2C \equiv CH$ | $CH_3$ | H | F | H | H | H | H | O | O | |
| 130 | H | $-OCH_2C \equiv CH$ | $CH_3$ | H | F | H | H | H | H | S | O | 154 |
| 131 | H | $NO_2$ | Cl | H | H | H | H | H | H | O | O | 168 |
| 132 | H | $NO_2$ | Cl | H | H | H | H | H | H | S | O | 92 |
| 133 | H | $NO_2$ | Cl | H | F | H | H | H | H | S | O | |
| 134 | H | $-(OCH_2CH_2-)_2OC_2H_5$ | Cl | H | F | H | H | H | H | S | O | |
| 135 | H | $-SCH_2COOCH_3$ | Cl | H | F | H | H | H | H | S | O | |
| 136 | H | $-(CH_2-)_7CH_3$ | Cl | H | H | H | H | H | H | S | O | 122 |
| 137 | H | H | CN | H | H | H | H | H | H | S | O | 185 |
| 138 | H | Cl | Cl | H | H | H | H | H | H | S | O | 113 |
| 139 | H | H | $NO_2$ | H | H | H | H | H | H | S | O | 201 |

Tabelle 1 - Fortsetzung

| Bsp.-Nr. | $R^1$ | $R^2$ | $R^3$ | $R^4$ | $R^5$ | $R^6$ | $R^7$ | $R^8$ | $R^9$ | Q | m | Schmelzpunkt/°C |
|---|---|---|---|---|---|---|---|---|---|---|---|---|
| 140 | H | $-OCH_2C{\equiv}CH$ | $OCH_3$ | H | H | H | H | H | H | S | 0 | 170 |
| 141 | H | $OCH_3$ | CN | H | H | H | H | H | H | S | 0 | 98 |
| 142 | H | -N-CO-O-<br>\|<br>$CH_2C{\equiv}CH$ | | H | H | H | H | H | H | S | 0 | 183 |
| 143 | H | $-(OCH_2CH_2-)_2OC_2H_5$ | Cl | H | F | H | H | H | H | O | 0 | |

Ausgangsstoffe der Formel (II)

Beispiel (II-1)

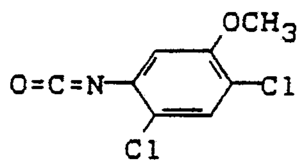

Zu 33 ml einer 1,93 M-Lösung von Phosgen in Toluol (= 63 mmol Phosgen) wird bei -10 °C langsam eine Lösung von 6 g (31 mmol) 2,4-Dichlor-5-methoxanilin in 100 ml Toluol getropft. Nach beendeter Zugabe läßt man auf Raumtemperatur kommen, erhitzt langsam zum Sieden und rührt dann zwei Stunden bei Siedetemperatur nach. Nach Ausblasen von überschüssigem Phosgen wird eingedampft.

Man erhält 5,7 g (84 % der Theorie) 2,4-Dichlor-5-methoxy-phenylisocyanat vom Schmelzpunkt 142 °C.

Beipiel (II-2)

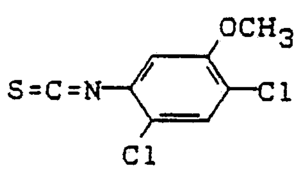

Zur Suspension von 1,53 ml (20 mmol) Thiophosgen in 20 ml eiskaltem Wasser werden unter gutem Rühren bei 0 °C bis 5 °C langsam 2,88 g (15 mmol) 2,4-Dichlor-5-methoxyanilin in 20 ml Chloroform getropft. Man läßt auf Raumtemperatur kommen, trennt die organische Phase ab, verdünnt mit 100 ml Toluol und destilliert bei Normaldruck überschüssiges Thiophosgen und Lösungsmittel ab. Der Rückstand wird mit 50 ml Toluol verdünnt, nach Trocknen über Natriumsulfat wird eingedampft.

Man erhält 3,4 g (97 % der Theorie) 2,4-Dichlor-5-methoxy-phenylisothiocyanat vom Schmelzpunkt 47 °C.

Analog Beispiel (II-1) bzw. (II-2) können die in der nachstehenden Tabelle 2 aufgeführten Verbindungen der allgemeinen Formel (II) hergestellt werden:

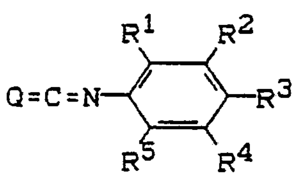

(II)

Tabelle 2: Beispiele für Verbindungen der Formel (II)

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-3 | H | H | Br | H | H | O | Fp. 44 |
| II-4 | H | H | Br | H | H | S | Fp. 61 |
| II-5 | H | Cl | $CF_3$ | H | H | O | Fp. 24 |
| II-6 | H | Cl | $CF_3$ | H | H | S | |
| II-7 | H | $CF_3$ | H | H | H | S | Kp: 208 |
| II-8 | H | $CF_3$ | H | H | H | O | $Kp_{11}$: 54 |
| II-9 | H | H | $OC_2H_5$ | H | H | O | |
| II-10 | H | H | $OC_2H_5$ | H | H | S | Kp: 61 |
| II-11 | H | Cl | $CH_3$ | H | H | O | $Kp_3$: 107 |
| II-12 | H | Cl | $CH_3$ | H | H | S | $Kp_8$: 127 |
| II-13 | H | F | H | H | H | O | |
| II-14 | H | H | F | H | H | O | |
| II-15 | H | H | H | H | F | O | |
| II-16 | H | H | H | H | Cl | O | $Kp_{10}$: 84 |
| II-17 | H | H | Cl | H | H | O | Fp. 31 |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-18 | H | H | Cl | H | H | S | Fp. 44 |
| II-19 | H | $OCH_3$ | Cl | H | F | O | Fp. 47 |
| II-20 | H | $OCH_3$ | Cl | H | F | S | Fp. 66 |
| II-21 | H | H | $NO_2$ | H | H | O | |
| II-22 | H | H | $NO_2$ | H | H | S | Fp. 112 |
| II-23 | H | $OCH_3$ | H | H | H | O | $Kp_{10}$: 95 |
| II-24 | H | $OCH(CH_3)_2$ | Cl | H | Cl | O | |
| II-25 | H | $OCH(CH_3)_2$ | Cl | H | Cl | S | |
| II-26 | H | $OCH_2COOCH_3$ | Cl | H | Cl | O | |
| II-27 | H | $OCH_2COOCH_3$ | Cl | H | Cl | S | Fp. 110 |
| II-28 | H | $OCH_3$ | Cl | H | H | O | |
| II-29 | H | $OCH_3$ | Cl | H | H | S | Fp. 58 |
| II-30 | H | $OCH(CH_3)_2$ | Cl | H | H | O | |
| II-31 | H | $OCH(CH_3)_2$ | Cl | H | H | S | |
| II-32 | H | $OCH_2COOCH_3$ | Cl | H | H | S | Fp. 79 |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-33 | H | $OCH_2COOCH_3$ | Cl | H | H | O | |
| II-34 | H | $OCHCOOCH_3$<br>\|<br>$CH_3$ | Cl | H | Cl | O | |
| II-35 | H | $OCHCOOCH_3$<br>\|<br>$CH_3$ | Cl | H | Cl | S | Fp. 127 |
| II-36 | H | $OCHCOOCH_3$<br>\|<br>$CH_3$ | Cl | H | H | O | |
| II-37 | H | $OCHCOOCH_3$<br>\|<br>$CH_3$ | Cl | H | H | S | Fp. 71 |
| II-38 | H | $OCH_2CH=CH_2$ | Cl | H | H | O | |
| II-39 | H | $OCH_2CH=CH_2$ | Cl | H | H | S | |
| II-40 | H | $-NH-CO-CH_2-O-$ | | H | F | O | |
| II-41 | H | $-NH-CO-CH_2-O-$ | | H | F | S | Fp. 203 |
| II-42 | H | Cl | F | Cl | F | O | |
| II-43 | H | Cl | F | Cl | F | S | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-44 | H | $OCH_2CH=CH_2$ | Cl | H | Cl | O | |
| II-45 | H | $OCH_2CH=CH_2$ | Cl | H | Cl | S | |
| II-46 | H | $OCH_2C\equiv CH$ | Cl | H | Cl | O | |
| II-47 | H | $OCH_2C\equiv CH$ | Cl | H | Cl | S | Fp. 84 |
| II-48 | H | $OCH_2C\equiv CH$ | Cl | H | H | O | |
| II-49 | H | $OCH_2C\equiv CH$ | Cl | H | H | S | Fp. 72 |
| II-50 | H | $OCH_2C\equiv CH$ | Cl | H | F | O | |
| II-51 | H | $OCH_2C\equiv CH$ | Cl | H | F | S | Fp. 76 |
| II-52 | H | $OCHCOOCH_3$<br>$CH_3$ | Cl | H | F | O | |
| II-53 | H | $OCHCOOCH_3$<br>$CH_3$ | Cl | H | F | S | Fp. 91 |
| II-54 | H | $OCH_2COOCH_3$ | Cl | H | F | O | |
| II-55 | H | $OCH_2COOCH_3$ | Cl | H | F | S | Fp. 101 |
| II-56 | H | $OCH(CH_3)_2$ | Cl | H | F | O | |

<u>Tabelle 2</u> - Fortsetzung

| Beisp.-Nr. | $R^1$ | $R^2$ | $R^3$ | $R^4$ | $R^5$ | Q | Schmelzpunkt bzw. Siedepunkt /°C |
|---|---|---|---|---|---|---|---|
| II-57 | H | $OCH(CH_3)_2$ | Cl | H | F | S | |
| II-58 | H | $OCH(CH_3)_2$ | Br | H | F | O | |
| II-59 | H | $OCH(CH_3)_2$ | Br | H | F | S | |
| II-60 | H | $OC_2H_5$ | Cl | H | F | S | |
| II-61 | H | $OC_2H_5$ | Br | H | F | S | |
| II-62 | H | $OCH_2CH_2CH_3$ | Cl | H | F | S | |
| II-63 | H | $OCH_2CH(CH_3)_2$ | Cl | H | F | S | |
| II-64 | H | $OCHCH_2CH_3$<br>\|<br>$CH_3$ | Cl | H | F | S | |
| II-65 | H | $OCH_2CH{=}CH_2$ | Cl | H | F | O | |
| II-66 | H | $OCH_2CH{=}CH_2$ | Cl | H | F | S | |
| II-67 | H | $OCHCH{=}CH_2$<br>\|<br>$CH_3$ | Cl | H | F | S | |
| II-68 | H | $OCHC{\equiv}CH$<br>\|<br>$CH_3$ | Cl | H | F | S | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/° C |
|---|---|---|---|---|---|---|---|
| II-69 | H | $OCH_2CH=CH_2$ | Br | H | F | O | |
| II-70 | H | $OCH_2CH=CH_2$ | Br | H | F | S | |
| II-71 | H | $OCH_2C\equiv CH$ | Br | H | F | O | |
| II-72 | H | $OCH_2C\equiv CH$ | Br | H | F | S | |
| II-73 | H | $OCH_2COOC_2H_5$ | Cl | H | F | S | |
| II-74 | H | $OCH_2COOCH(CH_3)_2$ | Cl | H | F | S | |
| II-75 | H | $OCH_2COO$ 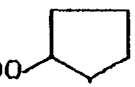 | Cl | H | F | O | |
| II-76 | H | $OCH_2COO$ 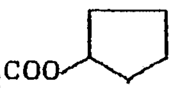 | Cl | H | F | S | |
| II-77 | H | $SCH_3$ | Cl | H | F | S | |
| II-78 | H | $OCH_2COOC_5H_{11}$ | Cl | H | F | S | |
| II-79 | H | $OCH_2COOCH_2$ 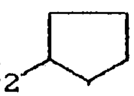 | Cl | H | F | O | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/° C |
|---|---|---|---|---|---|---|---|
| II-80 | H | $OCH_2COOCH_2$- 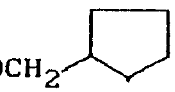 | Cl | H | F | S | |
| II-81 | H | $SCH_2COOCH_2$- 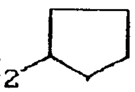 | Cl | H | F | O | |
| II-82 | H | $SC_2H_5$ | Cl | H | F | S | |
| II-83 | H | $SCH_2COOC_5H_{11}$ | Cl | H | F | O | |
| II-84 | H | $SCH_2COOC_5H_{11}$ | Cl | H | F | S | |
| II-85 | H | $SCH(CH_3)_2$ | Cl | H | F | O | |
| II-86 | H | $SCH_2CH=CH_2$ | Cl | H | F | O | |
| II-87 | H | $SCH_2CH=CH_2$ | Cl | H | F | S | |
| II-88 | H | $SCH_2C≡CH$ | Cl | H | F | S | |
| II-89 | H | $SCH_2C≡CH$ | Cl | H | F | O | |
| II-90 | H | $SCH_2COO$- 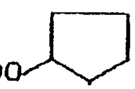 | Cl | H | F | O | |
| II-91 | H | $SC_4H_9$ | Cl | H | F | O | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-92 | H | $SCH_2COO$ 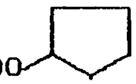 | Cl | H | F | S | |
| II-93 | H | $SCH_2CH{=}CHCH_3$ | Cl | H | F | S | |
| II-94 | H | $SCH_2COOCH_2$ 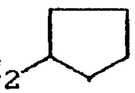 | Cl | H | F | S | |
| II-95 | H | $OCH_2CON(CH_3)_2$ | Cl | H | F | S | |
| II-96 | H | 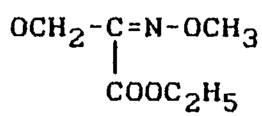 | Cl | H | F | O | |
| II-97 | H | 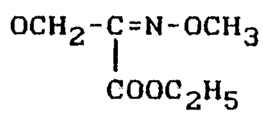 | Cl | H | F | S | |
| II-98 | H | 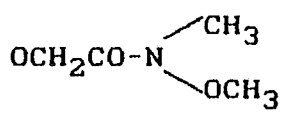 | Cl | H | F | S | |
| II-99 | H | 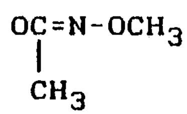 | Cl | H | F | S | |
| II-100 | H | $OCH_2CH{=}N{-}OCH_3$ | Cl | H | F | S | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-101 | H | $OCHCH=N-OC_2H_5$<br>\|<br>$CH_3$ | Cl | H | F | S | |
| II-102 | H | $OCH_2CH=N-OCH_2CH=CH_2$ | Cl | H | F | S | |
| II-103 | H | $OCH_2C=N-OCH_3$<br>\|<br>$CH_3$ | Cl | H | F | S | |
| II-104 | H | $OCH_2C=N-OCH_2CH=CH_2$<br>\|<br>$CH_3$ | Cl | H | F | S | |
| II-105 | H | $OCH_2CH=N-OCH_2COOCH_3$ | Cl | H | F | S | |
| II-106 | H | $OCH_2C=N-OCH_2COOCH_3$<br>\|<br>$CH_3$ | Cl | H | F | S | |
| II-107 | H | $SCH_3COOCH_2CH=CH_2$ | Cl | H | F | S | |
| II-108 | H | $SCH_2COOCH_2C\equiv CH$ | Cl | H | F | S | |
| II-109 | H | $OCHF_2$ | Cl | H | F | S | |
| II-110 | H | $OCF_2CHFCl$ | Cl | H | F | S | |

Tabelle 2 - Fortsetzung

| Beisp.-Nr. | $R^1$ | $R^2$ | $R^3$ | $R^4$ | $R^5$ | Q | Schmelzpunkt bzw. Siedepunkt/°C |
|---|---|---|---|---|---|---|---|
| II-111 | H | $OCH_2-\underset{\mid \atop Cl}{C}=CH_2$ | Cl | H | F | S | |
| II-112 | H | $OCF_2CHF_2$ | Cl | H | F | S | |
| II-113 | H | $OCH_2CH=CHCl$ | Cl | H | F | S | |
| II-114 | H | $OCF_2Cl$ | Cl | H | F | S | |

Anwendungsbeispiel

In dem folgenden Anwendungsbeispiel wird die nachstehend aufgeführte Verbindung als Vergleichssubstanz herangezogen:

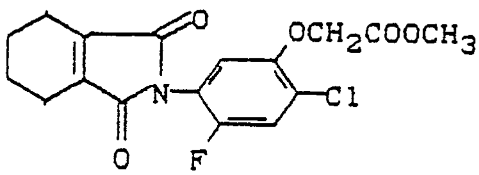

2-(4-Chlor-2-fluor-5-methoxycarbonylmethoxy-phenyl)-4,5,6,7-tetrahydro-2H-isoindol-1,3-dion (bekannt aus EP-A 83 055).

Beispiel A

Post-emergence-Test

Lösungsmittel: 5 Gewichtsteile Aceton Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad lder Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigt beispielsweise die gemäß Herstellungsbeispiel (13) erhaltene Verbindung bei besserer Verträglichkeit für Kulturpflanzen, wie z. B. Weizen und Mais, bei der Bekämpfung von Unkräutern, wie z. B. Galinsoga, Polygonum, Portulak, Sinapis und Veronica, deutlich stärkere Wirkung als die Vergleichssubstanz (A). Auch die gemäß Herstellungsbeispiel (15) erhaltene Verbindung zeigt gegen eine Reihe von Unkräutern, wie z. B. Galinsoga, Polygonum, Portulak, Sinapis, Veronica, Digitaria, und Setaria, erheblich stärkere Wirkung als die Vergleichssubstanz (A).

Darüber hinaus zeigen beispielsweise die Verbindungen gemäß der Herstellungsbeispiel 44, 47, 53, 54, 56, 57, 58, 65 und 109 ebenso eine bessere Nutzpflanzenverträglichkeit und eine bessere herbizide Wirkung gegen Unkräuter als die Vergleichssubstanz (A).

**Ansprüche**

1. Anellierte (Thio)Hydantoine der Formel (I)

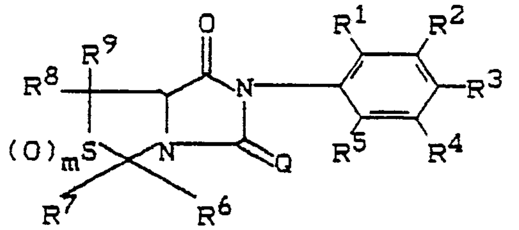

(I)

in welcher

m für die Zahlen 0, 1 oder 2 steht,

$R^1$ für Wasserstoff oder Halogen steht,

$R^2$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht,

$R^3$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht oder die beiden Reste

$R^2$ und $R^3$ zusammen für die Gruppierung $-X-A-(CO)_n-Y-$stehen, worin

n für die Zahlen 0 oder 1 steht,

A für eine direkte Bindung oder für geradkettiges oder verzweigtes und gegebenenfalls durch Halogen substituiertes Alkandiyl steht,

X für Sauerstoff oder Schwefel steht und

Y für Sauerstoff, Schwefel oder die Gruppierung $N-R^{10}$ steht, worin

$R^{10}$ für Wasserstoff oder gegebenenfalls substituiertes Alkyl, Alkenyl oder Alkinyl steht,

in welcher weiter

$R^4$ für Wasserstoff oder Halogen steht,

$R^5$ für Wasserstoff oder Halogen steht,

$R^6$ für Wasserstoff, gegebenenfalls substituiertes Alkyl oder gegebenenfalls substituiertes Phenyl oder Naphthyl steht,

$R^7$ für Wasserstoff oder Alkyl steht,

$R^8$ für Wasserstoff oder Alkyl steht,

$R^9$ für Wasserstoff oder Alkyl steht und

Q für Sauerstoff oder Schwefel steht

-mit der Maßgabe, daß

1. wenigstens einer der Reste $R^1$ bis $R^9$ von Wasserstoff verschieden ist,

2. $R^3$ nur dann für Methyl, Methoxy oder Chlor steht, wenn wenigstens einer der Reste $R^1$, $R^2$, $R^4$ und $R^5$ von Wasserstoff verschieden ist und

3. die Reste $R^2$ und $R^4$ nur dann gleichzeitig für Chlor stehen, wenn wenigstens einer der Reste $R^1$, $R^3$ und $R^5$ von Wasserstoff verschieden ist.

2. Anellierte (Thio)Hydantoine der Formel (I) gemäß Anspruch 1,

in welcher

m für 0, 1 oder 2 steht,

$R^1$ für Wasserstoff, Fluor, Chlor oder Brom steht,

$R^2$ für Wasserstoff, Nitro, Cyano, Fluor, Chlor oder Brom steht oder für einen gegebenenfalls durch Fluor, Chlor, Brom, Cyano, $C_3$-$C_6$-Cycloalkyl (welches gegebenenfalls durch Fluor, Chlor, Brom, Methyl und/oder Ethyl substituiert ist), $C_1$-$C_6$-Alkoxy, $C_1$-$C_4$-Alkoxy-$C_1$-$C_4$-alkoxy, $C_1$-$C_6$-Alkylthio, $C_1$-$C_6$-Alkylsulfinyl, $C_1$-$C_6$-Alkylsulfonyl, $C_1$-$C_4$-Alkylthio-$C_1$-$C_4$-alkoxy, $C_1$-$C_4$-Alkylsulfonyl-$C_1$-$C_4$-alkoxy, $C_3$-$C_6$-Alkenyloxy, $C_3$-$C_6$-Alkinyloxy, $C_3$-$C_6$-Alkenylthio, $C_3$-$C_6$-Alkinylthio, Carboxy, $C_1$-$C_6$-Alkoxy-carbonyl, $C_3$-$C_6$-Alkenyloxy-carbonyl, $C_3$-$C_6$-Alkinyloxy-carbonyl, $C_3$-$C_6$-Cycloalkyloxy-carbonyl, $C_3$-$C_6$-Cycloalkenyloxy-carbonyl, $C_3$-$C_6$-Cycloalkyl-$C_1$-$C_4$-alkoxy-carbonyl, $C_3$-$C_6$-Cycloalkenyl-$C_1$-$C_4$-alkoxy-carbonyl, $C_1$-$C_4$-Alkoxy-$C_1$-$C_4$-alkoxy-carbonyl, $C_1$-$C_4$-Alkylthio-$C_1$-$C_4$-alkoxy-carbonyl, Benzyloxy-$C_1$-$C_4$-alkoxy-carbonyl, $C_1$-$C_4$-Alkoxy-carbonyl-$C_1$-$C_4$-alkoxy-carbonyl, $C_1$-$C_4$-Alkylamino-carbonyl-$C_1$-$C_4$-alkoxy-carbonyl, Di-($C_1$-$C_4$-alkyl)-amino-carbonyl-$C_1$-$C_4$-alkoxy-carbonyl, $C_1$-$C_4$-Alkoxy-$C_1$-$C_4$-alkyl-amino-carbonyl, $C_1$-$C_4$-Alkoxy-imino-$C_1$-$C_4$-alkyl,

$C_3$-$C_4$-Alkenyloxy-imino-$C_1$-$C_4$-alkyl, $C_1$-$C_4$-Alkoxy-carbonyl-$C_1$-$C_4$-alkoxy-imino-$C_1$-$C_4$-alkyl, $C_1$-$C_6$-Alkylthio-carbonyl, $C_3$-$C_6$-Alkenylthio-carbonyl, $C_3$-$C_6$-Alkynlthio-carbonyl, Carbamoyl, $C_1$-$C_6$-Alkylamino-carbonyl, $C_3$-$C_6$-Alkenylamino-carbonyl, $C_3$-$C_6$-Alkinylamino-carbonyl, Di-($C_1$-$C_4$-alkyl)-amino-carbonyl, Di-($C_3$-$C_4$-alkenyl)-amino-carbonyl und/oder Di-($C_3$-$C_4$-alkinyl)-amino-carbonyl substituierten Rest aus der Reihe $C_1$-$C_6$-Alkyl, $C_1$-$C_6$-Alkoxy, $C_1$-$C_6$-Alkylthio und $C_1$-$C_6$-Alkylamino oder für einen gegebenenfalls durch Fluor und/oder Chlor substituierten Rest aus der Reihe $C_3$-$C_6$-Alkenyl, $C_3$-$C_5$-Alkinyl, $C_3$-$C_6$-Alkenyloxy, $C_3$-$C_6$-Alkinyloxy, $C_3$-$C_6$-Alkenylthio, $C_3$-$C_6$-Alkinylthio, $C_1$-$C_6$-Alkylsulfinyl und $C_1$-$C_6$-Alkylsulfonyl steht,

$R^3$ für Wasserstoff, Fluor, Chlor, Brom, Nitro, Cyano oder für einen gegebenenfalls durch Fluor und/oder Chlor substituierten Rest aus der Reihe $C_1$-$C_4$-Alkyl, $C_3$-$C_4$-Alkenyl, $C_3$-$C_4$-Alkinyl, $C_1$-$C_4$-Alkoxy, $C_3$-$C_4$-Alkenyloxy, $C_3$-$C_4$-Alkinyloxy, $C_1$-$C_4$-Alkylthio, $C_3$-$C_4$-Alkenylthio, $C_3$-$C_4$-Alkinylthio, $C_1$-$C_4$-Alkylsulfinyl, $C_1$-$C_4$-Alkylsulfonyl und $C_1$-$C_4$-Alkylamino steht, oder die beiden Reste

$R^2$ und $R^3$ zusammen für die Gruppierung -X-A-$(CO)_n$-Y-steht, worin

n für die Zahlen 0 oder 1 steht,

A für eine direkte Bindung oder für geradkettiges oder verzweigtes und gegebenenfalls durch Fluor und/oder Chlor substituiertes $C_1$-$C_4$-Alkandiyl steht,

X für Sauerstoff oder Schwefel steht und

Y für Sauerstoff, Schwefel oder die Gruppierung N-$R^{10}$ steht worin,

$R^{10}$ für Waserstoff oder gegebenenfals durch Fluor und/oder Chlor substituierte Reste aus der Reihe $C_1$-$C_4$-Alkyl, $C_3$-$C_4$-Alkenyl und $C_3$-$C_4$-Alkinyl steht,

in welcher weiter

$R^4$ für Wasserstoff, Fluor, Chlor oder Brom steht,

$R^5$ für Wasserstoff, Fluor, Chlor oder Brom steht,

$R^6$ für Wasserstoff, gegebenenfalls durch Fluor und/ oder Chlor substituiertes $C_1$-$C_6$-Alkyl oder gegebenenfalls durch Fluor, Chlor, Brom, Cyano, Nitro, $C_1$-$C_4$-Alkyl, Trifluormethyl, $C_1$-$C_4$-Alkoxy und/oder $C_1$-$C_2$-Fluoralkoxy substituiertes Phenyl oder Naphthyl steht,

$R^7$ für Wasserstoff oder $C_1$-$C_4$-Alkyl steht,

$R^8$ für Wasserstoff oder $C_1$-$C_4$-Alkyl steht,

$R^9$ für Wasserstoff oder $C_1$-$C_4$-Alkyl slteht und

Q für Sauerstoff oder Schwefel steht,

-mit der Maßgabe, daß

1. wenigstens einer der Reste $R^1$ bis $R^9$ von Wasserstoff verschieden ist,

2. $R^3$ nur dann für Methyl, Methoxy oder Chlor steht, wenn wenigstens einer der Reste $R^1$, $R^2$, $R^4$ und $R^5$ von Wasserstoff verschieden ist und

3. die Reste $R^2$ und $R^4$ nur dann gleichzeitig für Chlor stehen, wenn wenigstens einer der Reste $R^1$, $R^3$ und $R^5$ von Wasserstoff verschieden ist.

3. Anellierte (Thio)Hydantoine der Formel (I) gemäß Anspruch 1, in welcher

m für Null steht,

$R^1$ für Wasserstoff steht,

$R^2$ für Wasserstoff, Nitro, Cyano, Fluor, Chlor oder Brom oder für einen gegebenenfalls durch Fluor, Chlor, Cyclopropyl (welches durch Chlor und/oder Methyl substituiert sein kann), $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, Carboxy, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl, Di-($C_1$-$C_2$-alkyl)-aminocarbonyl, $C_1$-$C_2$-Alkoxy-$C_1$-$C_2$-alkyl-amino-carbonyl, $C_3$-$C_4$-Alkenyloxy-imino-$C_1$-$C_2$-alkyl, $C_1$-$C_2$-Alkoxy-imino-$C_1$-$C_2$-alkyl, $C_1$-$C_2$-Alkoxy-carbonyl-$C_1$-$C_2$-alkoxy-imino-$C_1$-$C_2$-alkyl substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Methyl oder Trifluormethyl steht, oder die beiden Reste

$R^2$ und $R^3$ zusammen für die Gruppierung -X-A-$(CO)_n$-Y-stehen, worin

n für die Zahlen 0 oder 1 steht,

A für eine direkte bindung oder für Methylen, Ethylen, Propylen oder Butylen steht

X für Sauerstoff steht,

Y für Sauerstoff die Gruppierung N-$R^{10}$ steht, worin

$R^{10}$ für Wasserstoff, $C_1$-$C_4$-Alkyl, $C_3$-$C_4$-Alkenyl oder $C_3$-$C_4$-Alkinyl steht,

in welcher weiter

$R^4$ für Wasserstoff steht,

$R^5$ für Wasserstoff, Fluor Chlor steht,

$R^6$ für Wasserstoff, $C_1$-$C_4$-Alkyl oder für gegebenenfalls durch $C_1$-$C_2$-Alkoxy oder Nitro substituiertes Phenyl steht sowie

$R^7$, $R^8$ und $R^9$ unabhängig voneinander für Wasserstoff, Methyl oder Ethyl stehen und

Q für Sauerstoff oder Schwefel steht,

-mit der Maßgabe, daß

1. wenigstens einer der Reste $R^2$, $R^3$, $R^5$, $R^6$,.$R^7$, $R^8$ und $R^9$ von Wasserstoff verschieden ist und
2. $R^3$ nur dann für Chlor steht, wenn wenigstens einer der Reste $R^2$ und $R^5$ von Wasserstoff verschieden ist.

4. Anellierte (Thio)Hydantoine der Formel (I) gemäß Anspruch 1, in welcher

m für Null steht,

$R^1$ für Wasserstoff steht,

$R^2$ für Wasserstoff, Fluor, Chlor oder Brom oder für einen gegebenenfalls durch Fluor, Chlor, Cyclopropyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_3$-$C_4$-Alkenyloxy, $C_3$-$C_4$-Alkinyloxy, $C_3$-$C_4$-Alkenylthio, $C_3$-$C_4$-Alkinylthio, $C_1$-$C_4$-Alkoxy-carbonyl, $C_3$-$C_4$-Alkenyloxy-carbonyl, $C_3$-$C_4$-Alkinyloxy-carbonyl, $C_3$-$C_6$-Cycloalkyloxy-carbonyl, $C_3$-$C_6$-Cycloalkyl-$C_1$-$C_2$-alkoxy-carbonyl, $C_1$-$C_4$-Alkoxy-$C_1$-$C_2$-alkoxy-carbonyl, $C_1$-$C_4$-Alkylthio-$C_1$-$C_2$-alkoxy-carbonyl, $C_1$-$C_4$-Alkoxy-carbonyl-$C_1$-$C_2$-alkoxy-carbonyl oder $C_1$-$C_4$-Alkylamino-carbonyl substituierten Rest aus der Reihe $C_1$-$C_4$-Alkyl, $C_1$-$C_4$-Alkoxy, $C_1$-$C_4$-Alkylthio und $C_1$-$C_4$-Alkylamino oder für einen gegebenenfalls durch Fluor und/oder Chlor substituierten Rest aus der Reihe $C_3$-$C_4$-Alkenyloxy, $C_3$-$C_4$-Alkinyloxy, $C_3$-$C_4$-Alkenylthio und $C_3$-$C_4$-Alkinylthio steht,

$R^3$ für Wasserstoff, Fluor, Chlor, Brom, Methyl oder Trifluormethyl steht, oder die beiden Reste

$R^2$ und $R^3$ zusammen für die Gruppierung -X-A-$(CO)_n$-Y-stehen, worin

n für die Zahlen 0 oder 1 steht,

A für Methylen, Ethylen, Propylen oder Butylen steht,

X für Sauerstoff steht,

Y für Sauerstoff oder die Gruppierung N-$R^{10}$ steht, worin

$R^{10}$ für Wasserstoff, $C_1$-$C_4$-Alkyl, $C_3$-$C_4$-Alkenyl oder $C_3$-$C_4$-Alkinyl steht,

in welcher weiter

$R^4$ für Wasserstoff steht,

$R^5$ für Wasserstoff oder Fluor steht,

$R^6$, $R^7$, $R^8$ und $R^9$ für Wasserstoff stehen und

Q für Sauerstoff oder Schwefel steht,

-mit der Maßgabe, daß

1. wenigstens einer Reste $R^2$, $R^3$ und $R^5$ von Wasserstoff verschieden ist und
2. $R^3$ nur dann für Chlor steht, wenn wenigstens einer der Reste $R^2$ und $R^5$ von Wasserstoff verschieden ist,

5. Verfahren zur Herstellung der anellierten (Thio)-Hydantoine der Formel (I)

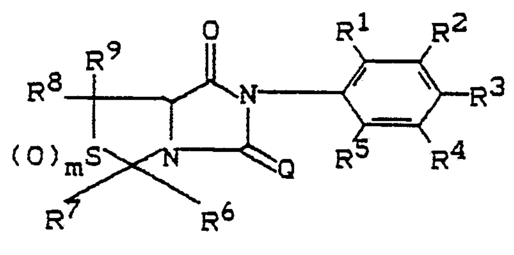

(I)

in welcher

m für die Zahlen 0, 1 oder 2 steht,

$R^1$ für Wasserstoff oder Halogen steht,

$R^2$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht,

$R^3$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht oder die beiden Reste

$R^2$ und $R^3$ zusammen für die Gruppierung -X-A$(CO)_n$-Y-stehen, worin

n für die Zahlen 0 oder 1 steht,

A für eine direkte Bindung oder für geradkettiges oder verzweigtes und gegebenenfalls durch Halogen substituiertes Alkandiyl steht,

X für Sauerstoff oder Schwefel steht und

Y für Sauerstoff, Schwefel oder die Gruppierung N-$R^{10}$ steht, worin

$R^{10}$ für Wasserstoff oder gegebenenfalls substituiertes Alkyl, Alkenyl oder Alkinyl steht,

in welcher weiter

$R^4$ für Wasserstoff oder Halogen steht,

$R^5$ für Wasserstoff oder Halogen steht,

$R^6$ für Wasserstoff, gegebenenfalls substituiertes Alkyl oder gegebenenfalls substituiertes Phenyl oder Naphthyl steht,

$R^7$ für Wasserstoff oder Alkyl steht,

$R^8$ für Wasserstoff oder Alkyl steht,

$R^9$ für Wasserstoff oder Alkyl steht und

Q für Sauerstoff oder Schwefel steht

-mit der Maßgabe, daß

1. wenigstens einer der Reste $R^1$ bis $R^9$ von Wasserstoff verschieden ist,

2. $R^3$ nur dann für Methyl, Methoxy oder Chlor steht, wenn wenigstens einer der Reste $R^1$, $R^2$, $R^4$ und $R^5$ von Wasserstoff verschieden ist und

3. die Reste $R^2$ und $R^4$ nur dann gleichzeitig für Chlor stehen, wenn wenigstens einer der Reste $R^1$, $R^3$ und $R^5$ von Wasserstoff verschieden ist,

dadurch gekennzeichnet, daß man Aryliso(thio)-cyanate der Formel (II)

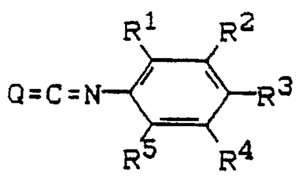

(II)

in welcher

$R^1$, $R^2$, $R^3$, $R^4$, $R^5$ und Q die oben angegebenen Bedeutungen haben,

mit Thiazolidincarbonsäure(ester)n der allgemeinen Formel (III)

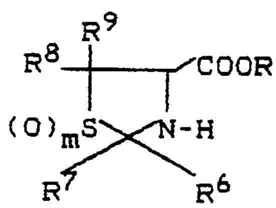

(III)

in welcher

m, $R^6$, $R^7$, $R^8$ und $R^9$ die oben angegebenen Bedeutungen haben und

R für Wasserstoff oder Alkyl steht,

gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einem anellierten (Thio)-Hydantoin der Formel (IA)

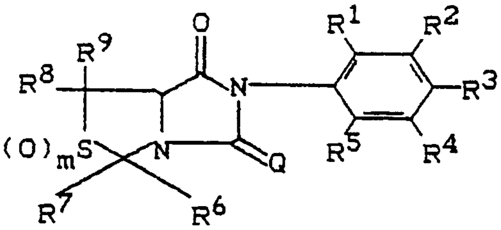

(Ia)

in welcher

m für die Zahlen 0, 1 oder 2 steht,

$R^1$ für Wasserstoff oder Halogen steht,

$R^2$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht,

$R^3$ für Wasserstoff, Halogen, Nitro, Cyano oder einen gegebenenfalls substituierten Rest aus der Reihe Alkyl, Alkenyl, Alkinyl, Alkoxy, Alkenyloxy, Alkinyloxy, Alkylthio, Alkenylthio, Alkinylthio, Alkylsulfinyl, Alkylsulfonyl und Alkylamino steht oder die beiden Reste

$R^2$ und $R^3$ zusammen für die Gruppierung $-X-A-(CO)_n-Y-$stehen, worin

n für die Zahlen 0 oder 1 steht,

A für eine direkte Bindung oder für geradkettiges oder verzweigtes und gegebenenfalls durch Halogen substituiertes Alkandiyl steht,

X für Sauerstoff oder Schwefel steht und

Y für Sauerstoff, Schwefel oder die Gruppierung $N-R^{10}$ steht, worin

$R^{10}$ für Wasserstoff oder gegebenenfalls substituiertes Alkyl, Alkenyl oder Alkinyl steht,

in welcher weiter

$R^4$ für Wasserstoff oder Halogen steht,

$R^5$ für Wasserstoff oder Halogen steht,

$R^6$ für Wasserstoff, gegebenenfalls substituiertes Alkyl oder gegebenenfalls substituiertes Phenyl oder Naphthyl steht,

$R^7$ für Wasserstoff oder Alkyl steht,

$R^8$ für Wasserstoff oder Alkyl steht,

$R^9$ für Wasserstoff oder Alkyl steht und

Q für Sauerstoff oder Schwefel steht.

7. Verfahren zur Bekämpfung von Unkräutern, dadurch gekennzeichnet, daß man anellierte (Thio)-Hydantoine der Formel (Ia) gemäß Anspruch 6 auf Unkräuter und/oder deren Lebensraum einwirken läßt.

8. Verwendung von anellierten (Thio)Hydantoinen der Formel (Ia) gemäß Anspruch 6 zur Bekämpfung von Unkräutern.

9. Verfahren zur Herstellung von herbiziden Mitteln, dadurch gekennzeichnet, daß man anellierte (Thio)-Hydantoine der Formel (Ia) gemäß Anspruch 6 mit Streckmitteln und/oder oberflächenaktiven Substanzen vermischt.

10. Verbindung der Formel (IIa)

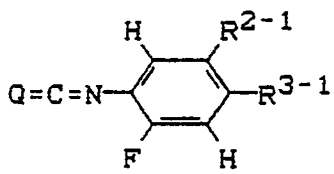

(IIa)

in welcher

Q für Sauerstoff oder Schwefel steht,

$R^{2-1}$ für einen gegebenenfalls substituierten rest aus der Reihe Alkoxy, Alkenyloxy, Alkinyloxy, Alkylthio, Alkenylthio steht und

$R^{3-1}$ für Halogen oder für gegebenenfalls substituiertes Alkyl steht

-mit Ausnahme der Verbindungen, bei denen $R^{2-1}$ Propoxy, Allyloxy, Propargyloxy oder Acetylmethoxy steht und gleichzeitig $R^{3-1}$ für Chlor steht.